# EUROPÄISCHE PATENTANMELDUNG

(11) **EP 2 695 612 A1**
(43) Veröffentlichungstag der Anmeldung: **12.02.2014**
(21) Anmeldenummer: 13177178.4
(22) Anmeldetag: 19.07.2013
(51) Int. Cl.: A61K 31/17, A61K 31/205, A61K 31/221, A61P 17/00

(54) **Verwendung von Wirkstoffkombinationen aus Harnstoff und Carnitin und/oder einem oder mehreren Acyl-Carnitinen zur Behandlung und Prophylaxe von Neurodermitis sowie des diabetischen Fußes**

(30) Priorität: 08.08.2012 DE 102012214038
(71) Anmelder: Beiersdorf AG, 20253 Hamburg (DE)
(72) Erfinder: Filbry, Alexander, 22459 Hamburg (DE); Kröpke, Rainer, 22869 Schenefeld (DE); Breitenbach, Ute, 22299 Hamburg (DE); Zelle, Dagmar, 21640 Bliedersdorf (DE)

(57) **Zusammenfassung**

Verwendung von Wirkstoffkombinationen aus Harnstoff und Carnitin und/oder einem oder mehreren Acyl-Carnitinen zur Prophylaxe und/oder Behandlung der Neurodermitis sowie des diabetischen Fußes.

## Beschreibung

Die vorliegende Erfindung betrifft die Verwendung von Wirkstoffkombinationen aus Harnstoff und Carnitin und/oder einem oder mehreren Acyl-Carnitinen zur Neurodermitis sowie des diabetischen Fußes bzw. zur Herstellung von pharmazeutischen Zubereitungen, die gegen Neurodermitis und/oder den diabetischen Fuß wirksam sind.

Die Bezeichnung Neurodermitis wurde ursprünglich als Sammelbegriff für stark juckende Dermatosen, bei denen eine Beteiligung des Nervensystems vermutet wurde, verwendet. Heute ist die Verwendung des Begriffs im Wesentlichen auf Neurodermitis constitutionalis (andere Namen: atopisches Ekzem, atopische Dermatitits, endogenes Ekzem, Neurodermitis atopica, Prurigo Besnier; besondere Erscheinungsformen bzw. Vorläuferformen: Milchschorf, Säuglingsekzem) beschränkt. Die Erkrankung wird heute als für die Betroffenen quälende und auch psychisch belastende Zivilisationskrankheit angesehen, und insbesondere ihre verbreiteten schweren, langdauernden Verlaufsformen im Kleinkind- und Kindesalter bereiten Eltern und Kinderärzten erhebliche Probleme.

Als mögliche Ursachen werden die verschiedensten Faktoren diskutiert. So wird die Beteiligung der Erbanlagen für die Entstehung der Neurodermitis genauso diskutiert wie Einflüsse der Umwelt. Als Umweltfaktoren, die krankheitsauslösend sein können, werden dabei insbesondere diskutiert: Luftschadstoffe, ein verändertes Wohnverhalten (zentralbeheizte Betonplatten-Bauten), vermehrte Allergen-Exposition (Haustiere, Hausstaubmilben) und möglicherweise Ernährungsgewohnheiten und Rauchgewohnheiten. Eine in letzter Zeit vermehrt diskutierte Erklärungsvariante ist die Infektionshypothese: Möglicherweise hat der Anstieg dieser atopischen Erkrankung auch damit zu tun, dass in den Industriestaaten bestimmte virale (Masern, Hepatitis) und bakterielle (Tbc) Infektionskrankheiten kaum mehr vorkommen. Dadurch ist die Th-1 getriebene Immunantwort (durch Th1-Zellen) unterfordert, und es entsteht ein relatives Übergewicht von Th2-Lymphozyten, die für den Ablauf dieser atopischen Erkrankungen kennzeichnend sind (siehe Deutsche Apothekerzeitung, 139. Jahrgang, Nr. 40 vom 7.10.1999, Seite 43-46). Nach aktuellen Erkenntnissen scheint beim aktiven atopischen Ekzem außerdem der grampositive Erreger Staphylococcus aureus eine besondere Rolle zu spielen. So ist dieser Keim das mikrobiologische Agens einer häufigen Komplikation der Neurodermitis, des impetiginisierenden atopischen Ekzems, das gekennzeichnet ist durch honiggelbe Krustenbildungen.

Zur Therapie der Neurodermitis kommen neben Versuchen, die Erkrankung durch Umstellung von Lebensweise und Ernährung positiv zu beeinflussen, cortisonhaltige Salben und Antibiotika zum Einsatz, wobei allerdings bei letzteren wegen zunehmender Resistenzen von Staphylococcus aureus gegen Penicillin und Erythromycin immer mehr auf teurere Antibiotika ausgewichen werden muß. Außerdem werden juckreizlindernde Antihistaminika (Wirkstoff: Clemastin, Promethazin, Hydroxyzin, Dimetinden, Doxylamin u. a.) eingesetzt.

Die Behandlungserfolge sind jedoch häufig nur enttäuschend. Es besteht daher ein dringender Bedarf nach einem neuen Mittel, das in der Lage ist, bei seiner Verwendung eine wirksame, nachhaltige Besserung von Neurodermitis-Erkrankungen bewirkt.

Es ist Aufgabe der vorliegenden Erfindung, diesen Bedarf zu stillen.

Die Problematik des diabetischen Fußes besteht in der Verminderung der natürlichen Funktionen, einhergehend mit allen Infektionsformen sowie der durch körperliche Nachteile entstandenen verminderten Pflege/Selbstpflegemöglichkeiten. Beim diabetischen Fuß treten spezifische Symptome an den Füßen auf: - Trockenheit der Haut - Verlust der Elastizität-Übermäßige Hornhautbildung - Dadurch gesamthaft Rißbildungen, Schrunden mit - gesteigerter Infektionsgefahr für Pilze und Bakterien - belastete Wundsituationen.

Betroffene sind häufig altersmäßig und krankheitsbedingt nicht mehr in der Lage, die Hygiene- und Therapievorschriften zur Minderung der latenten Gefahren zu erbringen. Ungeschulte Pflegepersonen sind durch den Anblick und die unästhetischen Begleitumstände häufig überfordert. Die sachgerechte Minderung von Risiken unterbleibt. - sekundär höhere Heil- und Behandlungskosten und Nachsorge- und Folgekosten werden provoziert.

Die Aufgabe wird durch die Verwendung von Wirkstoffkombinationen aus Harnstoff und Carnitin und/oder einem oder mehreren Acyl-Carnitinen zur Prophylaxe und/oder Behandlung der Neurodermitis sowie des diabetischen Fußes gelöst.

L-Carnitin [3-Hydroxy-4-(trimethylammonio)-buttersäurebetain], weist die Strukturformel 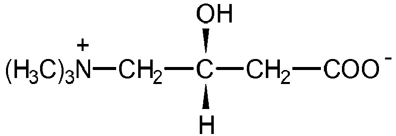 (Summenformel C₇H₁₅NO₃) auf.

Die L-Form des Carnitins ist in tierischen Geweben, insbesondere der gestreiften Muskulatur, weit verbreitet. Es dient im Fettsäure-Stoffwechsel als Überträger für Acylgruppen durch die Mitochondrien-Membran hindurch. Diese werden durch eine Acyltransferase von Acyl-Coenzym A auf die Hydroxygruppe des L-Carnitins übertragen. Der Transport von L-Carnitin und Acyl-L-Carnitin durch die Membran erfolgt durch Vermittlung eines Transportproteins (Translocase). Beide Entantiomere (D- und L-Form) sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden.

Erfindungsgemäß werden Acyl-Carnitine gewählt aus der Gruppe der Substanzen der folgenden allgemeinen Strukturformel 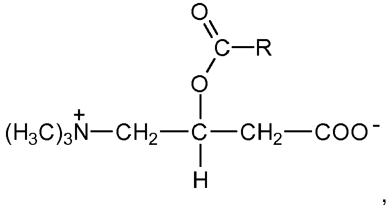 wobei R gewählt wird aus der Gruppe der verzweigten und unverzweigten Alkylreste mit bis zu 10 Kohlenstoffatomen. Bevorzugt sind Propionylcarnitin und, ganz besonders bevorzugt, Acetylcarnitin. Beide Entantiomere (D- und L-Form) sind vorteilhaft im Sinne der vorliegenden Erfindung zu verwenden. Es kann auch hier von Vorteil sein, beliebige Enantiomerengemische, beispielsweise ein Racemat aus D- und L-Form, zu verwenden.

Vorteilhaft enthalten die erfindungsgemäßen Zubereitungen 0,001 - 10 Gew.-% an Carnitin und/oder einem oder mehreren Acylcarnitinen, bezogen auf das Gesamtgewicht der Zubereitungen.

Kosmetische oder dermatologische Zubereitungen gemäß der Erfindung enthalten vorteilhaft 0,01 bis 60 Gew.-%, bevorzugt 0,1 bis 25 Gew.-%, insbesondere bevorzugt 1 bis 10 Gew.-% an Harnstoff, bezogen auf das Gesamtgewicht der Zubereitungen.

Erfindungsgemäß können Zubereitungen, welche die erfindungsgemäßen Wirkstoffkombinationen enthalten, übliche Antioxidantien eingesetzt werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Prophylaxe bzw. die kosmetische oder dermatologische Behandlung mit dem erfindungsgemäß verwendeten Wirkstoff bzw. mit den kosmetischen oder topischen dermatologischen Zubereitungen mit einem wirksamen Gehalt an erfindungsgemäß verwendetem Wirkstoff erfolgt in der üblichen Weise, und zwar dergestalt, dass der erfindungsgemäß verwendete Wirkstoff bzw. die kosmetischen oder topischen dermatologischen Zubereitungen mit einem wirksamen Gehalt an erfindungsgemäß verwendetem Wirkstoff auf die betroffenen Hautstellen aufgetragen wird.

Erfindungsgemäße Emulsionen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wäßrige Systeme bzw. Tensidzubereitungen zur Reinigung der Haut und der Haare einzufügen.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechenden Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Die erfindungsgemäßen kosmetischen und/oder dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden oder in den kosmetischen und dermatologischen Reinigungsprodukten.

Sofern die kosmetische oder dermatologische Zubereitung eine Lösung oder Lotion darstellt, können als Lösungsmittel verwendet werden: Wasser oder wäßrige Lösungen, ferner Öle, wie Triglyceride der Caprin- oder der Caprylsäure, vorzugsweise aber Rizinusöl, Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren, aber auch Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet, so wie bei alkoholischen Lösungsmitteln Wasser ein vorteilhafter weiterer Bestandteil sein kann.

Kosmetische und dermatologische Zubereitungen gemäß der Erfindung, auch z.B. zum Schutze der Haut vor UV-Strahlen, können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), Öl-in-Wasser-in-Öl (O/W/O), ein Gel, eine Hydrodispersion, eine lamellare Phase, eine flüssige isotrope Lösungsphase, eine micellare Phase, eine solide oder dispergierte ein- oder mehrfach hexagonale Phase, eine solide oder dispergierte ein- oder mehrfach kubische Phase, eine lyotrope Phase, eine kristalline Phase, einen festen Stift oder auch ein Aerosol darstellen.

Die erfindungsgemäßen Wirkstoffe können auch besonders vorteilhaft in Mikroemulsionen, beispielsweise wie in der deutschen Offenlegungsschrift DE-195 9 079 beschrieben, verwendet werden.

Die Ölphase der erfindungsgemäßen Zubereitungen wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Vorteilhaft beträgt der Gehalt an der Ölphase zwischen 1 und 50 Gew.-%, bezogen auf das Gesamtgewicht der Zubereitungen, bevorzugt 2,5 - 30 Gew.-%, insbesondere bevorzugt 5 - 15 Gew.-%.

Zur Anwendung werden die erfindungsgemäßen kosmetischen und/oder dermatologischen Formulierungen in der für Kosmetika und Dermatika üblichen Weise auf die Haut in ausreichender Menge aufgebracht.

Die folgenden Beispiele sollen die Verkörperungen der vorliegenden Erfindungen verdeutlichen.

| **Beispielrezeptur:** | **1** | **2** | **3** | **3** | **4** | **5** | **6** |
|---|---|---|---|---|---|---|---|
| Aluminumstärkeoctenylsuccinat | 1 | 1 | 1 | 1 | 1 | 1 | 1 |
| Caprylic/Capric Triglycerid | 4 | 4 | 4 | 4 | 1,5 | 1,5 | 1,5 |
| Carbomer/Natriumpolyacrylat | 0,2 | 0,2 | 0,2 | 0,2 | 0,2 | 0,2 | 0,2 |
| Carnitin | 1 | 0,5 | 2 | 4 | 1 | 0,5 | 4 |
| Cera Microcristallina + Medizinisches Weissöl | 4 | 4 | 4 | 4 | 2 | 2 | 2 |
| Cetylstearylalkohol | 3 | 3 | 3 | 3 | 2 | 2 | 2 |
| Cetylstearylalkohol + PEG-40 Rizinusöl + Natriumcetylstearvlalkohol | 3 | 3 | 3 | 3 | 3 | 3 | 3 |
| Ethyl hexylcocoat | 3 | 3 | 3 | 3 | 1,5 | 1,5 | 1,5 |
| Glycerin | 10 | 8 | 5 | 3 | 10 | 8 | 5 |
| Glycerylglucosid | | | | | 7 | 5 | 3 |
| Harnstoff | 10 | 5 | 20 | 40 | 10 | 20 | 40 |
| Hydrierte Kokosglyceride | 3 | 3 | 3 | 3 | 2 | 2 | 2 |
| lineares Silikonöl | 1 | 1 | 1 | 1 | 1 | 1 | 1 |
| Milchsäure | 0.9 | 0.9 | 0.9 | 0.9 | 0,9 | 0,9 | 0,9 |
| Natriumlactat | 5 | 5 | 5 | 5 | 5 | 5 | 5 |
| Octyldodecanol | 3 | 3 | 3 | 3 | 1.5 | 1,5 | 1,5 |
| Parfum | | | | | 0,4 | 0,4 | 0,4 |
| Phenoxyethanol | 0,8 | 0,8 | 0,8 | 0,8 | 0,8 | 0,8 | 0,8 |
| Sorbitanstearat | 1 | 1 | 1 | 1 | 1 | 1 | 1 |
| Wasser, dest. | Ad 100 | Ad 100 | Ad 100 | Ad 100 | Ad 100 | Ad 100 | Ad 100 |

## Patentansprüche

1. Verwendung von Wirkstoffkombinationen aus Harnstoff und Carnitin und/oder einem oder mehreren Acyl-Carnitinen zur Prophylaxe und/oder Behandlung der Neurodermitis sowie des diabetischen Fußes.

2. Verwendung nach Anspruch 1, **dadurch gekennzeichnet, daß** in den fertigen Zubereitungen Konzentrationen von 0,001 - 10 Gew.-% an Carnitin und/oder einem oder mehreren Acylcarnitinen vorliegen, bezogen auf das Gesamtgewicht der Zubereitungen.

3. Verwendung nach einem der vorstehenden Ansprüche, **dadurch gekennzeichnet, daß** in den fertigen Zubereitungen Konzentrationen von 0,01 bis 60 Gew.-%, bevorzugt 0,1 bis 40 Gew.-%, insbesondere bevorzugt 1 bis 20 Gew.-% an Harnstoff vorliegen, bezogen auf das Gesamtgewicht der Zubereitungen.
